## Europäisches Patentamt
## European Patent Office
## Office européen des brevets

(11) Publication number: **0 183 790 B1**

## EUROPEAN PATENT SPECIFICATION
### published in accordance with Art. 158(3) EPC

(45) Date of publication of patent specification: **11.09.91**

(51) Int. Cl.5: **C08L 75/04, B32B 27/40, B32B 27/06**

(21) Application number: **85902854.0**

(22) Date of filing: **21.05.85**

(86) International application number:
**PCT/US85/00946**

(87) International publication number:
**WO 85/05373 (05.12.85 85/26)**

(54) **MOISTURE VAPOR PERMEABLE MATERIALS CONTAINING SEGMENTED BLOCK MULTIPOLYMER.**

(30) Priority: **21.05.84 US 612330**

(43) Date of publication of application:
**11.06.86 Bulletin 86/24**

(45) Publication of the grant of the patent:
**11.09.91 Bulletin 91/37**

(84) Designated Contracting States:
**AT BE CH DE FR GB IT LI LU NL SE**

(56) References cited:
**EP-A- 0 107 915**
**GB-A- 2 073 219**
**US-A- 3 826 768**

(73) Proprietor: **Th. Goldschmidt AG**
**Goldschmidtstrasse 100 Postfach 101461**
**W-4300 Essen 1(DE)**

(72) Inventor: **WARD, Robert, S.**
**760 Tanglewood Lane**
**Lafayette, CA 94549(US)**

Rank Xerox (UK) Business Services

## Description

The present invention is directed to moisture vapor permeable materials in the form of nonporous uniform films or coatings less than 127 μm. These materials are segmented block multipolymers and may be used alone or in a mixture with a base polymer. Such materials are useful as membranes, wound dressings, surgical drapes and burn dressings and as coatings for fabrics for apparel and industrial uses.

Moisture vapor permeable backing materials having a moisture vapor permeable pressure sensitive adhesive are disclosed by Hodgson in US-Patent 3,645,835. The backing materials disclosed therein are synthetic polymers which are continuous and nonpermeable to liquid water. The adhesive materials are also permeable to moisture vapor. There is no disclosure of a discrete segmented block copolymer additive to either the backing material polymer or the adhesive material polymer to increase the moisture vapor transmission capability.

Other materials have been suggested for use as additives to polymers used in bio-medical devices, however, not for the purpose of enhancing moisture vapor transmission. For example, Nyilas in US-Patent 3,562,352 suggests cross-linked thermosetting polysiloxane-polyurethane block copolymers for use as blood contact surfaces of biomedical devices. The technique disclosed therein includes fabricating the entire bloodcontacting device from such block copolymers or coating such devices with the copolymers.

In GB-A-2 073 219 a blood-compatible polymer admixture is disclosed comprising at least 95 volume % of a base polymer and no greater than 5 volume % of a polymer additive comprising a poly-(dialkylsiloxane) segment chemically bonded to a polyurethane segment, said polymer additive being dispersed through said base polymer and being characterized by a $\gamma_c$ less than said base polymer, said polymer admixture being characterized by a $\gamma_c$ between about 10 and 35 dyne/cm. The polymer additive may be a ternary copolymer consisting of polydimethylsiloxane-polyethylene oxide-polyurethane or -polyureaurethane segments. Moisture vapor permeability is of no importance in this usage and therefore it is not mentioned in GB-A-2 073 219.

Minor amounts of block copolymers including segments of polydimethylsiloxane and blocks of polycarbonate, polystyrene, poly-(2,6-diphenyl-1,4-phenyleneoxide), and polyamide-imide have been blended with base homopolymers for modifying the surface properties of the homopolymers. Gaines et al. in US-Patent 3,961,122 suggest using such surface modified polymers as thin films while Gaines et al. in US-Patent 3,686,355 suggest a variety of uses, including bulk uses.

Flexible, soil resistant sheet material comprising a fibrous mat covered with a polyurethane composition is disclosed in US-Patent 4,233,359. The polyurethane composition contains 0.1 to 5 % of a surface active agent comprising a polyethylene oxide hydrophilic component and a hydrophobic component selected from polyalkylene oxides (except polyethylene oxides), aliphatic polyesters and mixtures thereof. No hydrophobic soft blocks such as polydialkylsiloxanes, are disclosed as part of the surface active agent.

Block copolymers useful in the treatment of fibrous materials to improve soil release properties are disclosed in US-Patent 3,565,845. The block copolymers are not mixed with a base polymer when used to treat the fibrous materials.

US-Patent 3,666,542 discloses a process for preparing a microporous structure wherein a dispersion of a polyurethane having hydrophilic polyoxyethylene segments in a solvent plus water is coated on a substrate and selectively evaporated. No nonporous uniform films or coatings are disclosed.

US-Patent 3,826,768 describes a process for preparing polyurethane compositions by dispersing a polyurethane made of selected polyols in water whereby a combination of three different surface active agents is used in order to enhance the stability of the dispersion, said combination of surface active agents is consisting of a) a sulfosuccinate type anionic surface active agent, b) a nonionic surface active agent having a HLB-value from 6 to 16 and c) a compound being obtained by reacting a sultone with a low molecular polyol and treating the resulting addition product with alkali. The dispersions are suitable for coating fabrics and the like.

EP-A-0 107 915 is concerning a moisture vapour permeable, adhesive surgical dressing which comprises a continuous film of a hydrophilic polymer which has a moisture vapour permeability which is greater when in contact with water than when not in contact with water and which is attached around its edges to a water transmitting film so as to form a sealed portion into which exudate may pass from an exuding wound. The hydrophilic polymer may consist of a polyurethane prepared by reacting polyethylene glycol, polytetramethylene glycol, ethane diol and 4,4'-dicyclohexylmethane diisocyanate.

US-Patent 4,052,495 describes a method of obtaining release of polyurethane polymers from various substrates by adding siloxane-polyoxyalkylene copolymers comprising OH-groups to the reaction mixture comprising polyols and polyisocyanates.

It is an object of the invention to provide nonporous uniform films and coatings of segmented block copolymers which can be used as wound dressings or coatings for textiles with high impermeability to liquid water, high permeability to water vapor, high textile strength and elongation and high soil-release properties.

The present invention is directed to a moisture vapor permeable material in the form of a nonporous uniform film or coating of a thickness less than 127 μm, said film or coating is obtained by using a solution of the material in an organic solvent and evaporating the solvent, said material comprising a segmented block multipolymer and optionally a base polymer, said segmented multipolymer comprising an essentially linear hard segment copolymer chain characterized by at least one polyurethane or polyurethane urea hard segment and at least one copolymer soft block comprising polyoxytetramethylene oxide and polydialkylsiloxane as hydrophobic and polyethylene oxide as hydrophilic components.

The materials according to the present invention will usually be formed by admixing a base polymer and a segmented block multipolymer additive in a solution and casting the admixture as a film. Removing the solvent by evaporative procedures will result in a material having a high moisture vapor capability.

When used without a base polymer, the block multipolymer will usually be dissolved in a solvent, then cast as a film or coating. This method is particularly preferred for forming moisture vapor permeable coatings on textiles.

As used herein, the term "base polymer" will refer to the polymer whose moisture vapor transmission characteristics are modified by the block multipolymer additive. Such base polymers may be segmented or block copolymers, blends thereof, thermoplastic homopolymers, or homopolymer mixtures which may also contain plasticizing agents. For example, plasticized polyvinylchloride and polyethylene may be used as base polymers.

Typical base polymers which may be utilized according to the present invention include polyurethanes, polysulfones, polyesters, polyethylenes, polypropylenes, polystyrenes, poly(acrylonitrile-butadienestyrene), polybutadiene, polyisoprene, styrene-butadiene-styrene block copolymers, styrene-isoprene-styrene block copolymers, poly-(4-methylpentene),polyisobutylene, polymethylmethacrylate, polyvinylacetate, polyacrylonitrile, polyvinylchloride, polyethylene terephthalate, cellulose and its esters or derivatives.

The term "segmented" refers to the relatively short length of repeating units, e.g., less than about ten monomeric units, but preferably less than three monomeric units, typically alternating more than once, with structural formulas, such as ABAB.

A preferred class of base polymers includes the polyurethanes, including polyether urethane ureas, polyether urethanes and polyester urethanes. The polyurethanes or polyurethane ureas may be the reaction products of polymeric ether glycols and a diisocyanate, and a chain extending diamine or dihydroxy derivative. By the use of various types of isocyanates (e.g. aliphatic or aromatic), glycols (e.g. polyethylene, polypropylene, or polytetramethylene oxides), and chain extenders (aliphatic or aromatic), the structural properties of the base polymer may be varied depending upon the end use of the material.

A particularly preferred base polymer is a polyurethane urea formed from the polymerization of diphenylmethane diisocyanate (MDI), ethylene diamine (ED), and polytetramethylene oxide (PTMO).

Other base polymers include polyester-polyethers, polyesters, polyether-polyamides, polyamides, styrene-isoprenes, styrene butadienes, thermoplastic polyolefins, styrene-saturated olefins, copolyesters, ethylene vinyl acetate, ethylene ethyl acrylate, ionomers, thermoplastic polydienes.

The segmented block multipolymers according to the present invention comprise an essentially linear segmented copolymer chain characterized by at least one polyurethane or polyurethane urea hard segment and at least one copolymer soft block comprising polyoxytetramethylene oxide and polydialkylsiloxane as hydrophobic and polyethylene oxide as hydrophilic components. The hard segment may be relatively short in length, typically including from one to ten repeating units. Preferably, the hard block will be a homopolymer wherein the hard segments comprise urethane selected from the same polymers which comprise the preferred polyurethane urea base polymers. When the segmented block multipolymer is used as an additive the similarity of the urethane segments of the additive and the urethane of the base polymer will cause the additive to be at least partially compatible with the base polymer in the admixture.

The copolymer soft block(s) of the block multipolymer are comprising both hydrophobic and hydrophilic components. The hydrophobic components of the soft block are consisting of polytetraalkylene oxide and a polydialkylsiloxane. Techniques for forming siloxane copolymers are known, e.g., as described by Noll, Chemistry and Technology of Silicones (Academic Press, 1968), disclosure of which is incorporated by reference herein.

The hard segments preferably comprise condensation products of diphenylmethane diisocyanate (MDI) with a diamine or diol. A particularly preferred hard segment will contain hard segments of MDI and ethylene diamine.

A particularly preferred composition of the first class will comprise the following:

```
15 - 50 %   (by weight) hard segment
20 - 45 %   polyethylene oxide
            (1000 - 3000 mol. wt. (number avg.))
25 - 65 %   polytetramethylene oxide
     2 %    polyalkylsiloxane,
```

-----------------

```
        100 %
```

The amounts of each of the block multipolymer additives which will be added to the base polymer, when used, will depend upon the base polymer and additives which are used. Generally, the organic polymer additive will be added in an amount to achieve a silicone concentration of at least 0.5 % (by weight) in the admixture containing both base polymer and additive.

In the above concentration ranges, the additives according to the present invention will cause an increase of the moisture vapor transmission rate of the base polymer by about 10 to 500 %, when cast in films ranging in thickness from 12.7 $\mu$m [0.5 mil] to 50.8 $\mu$m [2.0 mil].

To cast the films, the base polymer will normally be dissolved in an appropriate solvent, such as dimethylacetamide (DMAC), dimethylformamide (DMF), toluene/isopropanol, tetrahydrofuran (THF). The additives will then be added and the film will be cast according to conventional procedures onto a support. Upon evaporation of the solvent a film will remain comprising materials according to the present invention. Similar methods may also be utilized to form thermoplastic films.

The base polymers utilized according to the present invention may be those conventionally known in the art, i.e. polyurethanes, polyetherurethaneureas, polyetherurethanes, polyesterurethanes, polyester/polyether, thermoplastic elastomers, many of which are commercially available as Estanes (B.F. Goodrich Company), Pellethane (Upjohn), Hytrel and Lycra Spandex (DuPont).

The segmented block multipolymers according to the present invention may be suitably formed of block copolymers of alternating hard segment and soft block components interlinked by chemical bond in accordance with the method set forth by Noshay and McGrath, Block Copolymers Overview and Critical Survey (Academic Press 1977). A suitable number of repeating units of each polymer of the hydrophobic and hydrophilic soft block components will typically be on the order of 1 to 100 units.

The procedure for preparation of the segmented block multipolymer additive may be performed by several procedures which differ in the degree to which the structure of the resultant product may be defined.

One procedure involves a coupling of two (or more) preformed blocks or segments which are prepared in separate reactions prior to the coupling reaction. This procedure involves a well defined structure if the coupling reaction precludes like blocks or segments from reacting with themselves, but only allows dissimilar blocks or segments to couple to one another.

Other coupling reactions may occur resulting in a less defined structure of the two preformed blocks or segments possess the ability (via the coupling reaction) to react with themselves as well as the dissimilar block or segment.

Additional coupling reactions may occur when a single (or more) preformed block or segment is coupled with a second block or segment created during the coupling reaction. In this case the initial length of the preformed block or segment is known, by virtue of their separate reaction used to prepare but the sequence of the distribution of the copolymer is not known exactly since both coupling and chain growth is possible in the reaction. Suitable methods of forming these and other such copolymers for use in the present invention are set forth in the aforementioned Noshay and McGrath publication.

A typical procedure is given below:

Charge the reactor with 9.75 g (0.015 moles) of a linear, difunctional, hydroxy terminated polytetramethylene oxide, 72 g (0.03 moles) of a linear, difunctional, hydroxy terminated polyethylene oxide/polydimethyl-siloxane block copolymer, and 400 ml of a 1 : 1 (V/V) mixture of dimethylsulfoxide and methyl, isobutyl ketone. Heat the mixture to 75 - 80°C and add 37.5 g (0.15 moles) diphenylmethane diisocyanate (MDI) dissolved in 100 ml of a 1 : 1 (V/V) dimethylsulfoxide methyl, isobutyl ketone solvent mixture. Maintain the 75 - 80°C temperature with agitation and a dry nitrogen purge for three hours. Cool the reaction mixture to 45°C, then add 9.5 g (0.106 moles) butanediol in 200 ml of the 1 : 1 (V/V) dimethylsulfoxide/methyl, isobutyl ketone solvent. Maintain the 45°C temperature for approximately 2 hours. Isolate the polymer via coagulation in water.

The above preferred additive will be used in the preferred base polymer, which is a low durometer polyurethaneurea based on diphenylmethane diisocyanate (MDI), ethylenediamine (ED) and polytetramethylene oxide (PTMO). A typical procedure is outlined below.

Charge a stirred, dry nitrogen purged reaction vessel with 8.60 g (0.034 moles) diphenylmethane diisocyanate, 50.0 g (0.025 moles) polytetramethylene oxide, and 100 ml anhydrous dimethylformamide at 45°C. Maintain the 45°C temperature for 1 hour, 15 minutes. Remove the heat, dilute the mixture with 200 additional ml anhydrous dimethylformamide, and add 0.54 g (0.009 moles) ethylene diamine incrementally over a 20 minute period.

Typically, the base polymer will be dissolved in the solvent, preferably dimethylacetamide, and the additives will be added to the solution based on the weight of the base polymer. The amount of solvent which is used will depend at least in part on the thickness of the desired film. The thicknesses of the films which are desired will depend upon the ultimate use to which the film will be utilized.

The compatibility between the base polymer and the organic polymer additive may be controlled by using a constant silicone soft segment and varying the chemistry of the remainder of the components of the admixture. Therefore, if the base polymer is Estane 5714, the additive may be a polymer containing a low molecular weight silicone with the remainder of the additive comprising MDI, butanediol, and PTMO. On the other hand, using the same silicone content in a copolymer or a polymer additive containing ethylene diamine, polybutylene adipate and MDI may give a different compatibility with the same Estane 5714 base polymer at equivalent concentrations. Therefore, the choice of the amounts and types of additives and base polymers which are utilized according to their present ivention may be readily determined to achieve the most effective MVTR according to the teachings of the present invention.

Wound dressings comprising a nonporous uniform film according to the present invention may be made which have high elongation and excellent tear propagation so that the films may be stretched over joints and limbs to conform to parts of the body, while retaining the capability of being intentionally torn or cut without propagating rips across the dressing. A particularly preferable wound dressing material may comprise a polyether/polyester elastomer commercially available under the name Hytrel (DuPont) as the base polymer.

Textiles coated with a material according to the present invention will have low permeability to liquid water, high permeability to water vapor, high tensile strength and elongation. Generally, fabrics will be coated with a composition comprising either a segmented block multipolymer or a mixture of a base polymer and block multipolymer additive by conventional coating techniques. The compositions according to the present invention are particularly advantageous as fabric coatings due to excellent adhesive properties, and lack of tackiness which allows the coated fabrics to be easily sewn. In addition, the viscosity of the bulk composition is in the range which permits easy handling for coating techniques. Finally, the coated fabrics have a pleasing texture and are more cloth-like in feel than conventional water impermeable synthetic materials which characteristically have boardy texture. While not intending to limit the invention by a particular theory, it is believed that commercially available water impermeable coated textiles are microporous while the coated textiles according to the present invention form a substantially solid sheet of water-vapor permeable material to give a pleasing cloth-like texture while improving the soil-release and liquid water impermeability. Coated fabrics according to the present invention are useful as rainwear, uphostery, shoe tops, tablecloths, tarpaulines, protective interliners for clothing proofed against chemical agents, and similar uses where liquid, e.g., water impermeability and water vapor permeability is desired.

Having described the preferred embodiments, the following examples are presented, but the invention is not intended to be limited thereto.

Determination of the moisture vapor transmission rate (MVTR)

Fisher/Payne Permeability cups were used to determine the moisture vapor transmission rate (MVTR) of the test specimens. Ten milliliters of distilled water was dispensed into the flanged cup. A disk of the sample was placed over the cup flange followed by a butyl rubber sealing gasket and a metal flat ring. The sample, gasket and ring were secured to the cup flange with three clamps with sufficient pressure to insure a tight seal.

The MVTR of the unsupported cast films were determined by the standard test conditions of ASTM E-96, procedure BW. This procedure involved testing the material at 23 ± 1°C and 50 ± 2 % relative humidity. The cup was inverted allowing the distilled water to cover the inner surface of the film. Air was continuously circulated over the face of the inverted cup at 152 m/min. [500 ft/min.].

The samples were conditioned for 24 hours at 23° C and 50 % RH prior to testing. The MVTR of five specimens from each cast film were measured. The assembled cups were placed in the test chambers for one hour. The temperature, air velocity and relative humidity were recorded. At the end of this time period, each cup was accurately weighed to 0.1 mg. The cups were inverted and returned to the test chamber for two hours and reweighed. The MVTR of each cup was calculated from equation (1).

$$MVTR = (g)(24 \text{ hr})/(t)(A)$$

where:

g = weight change during test (grams)
t = time of analysis (hour)
A = test area (cup mouth area) $(m^2)$

Each film was analyzed on three separate days to determine the reproducibility of the coating and testing methods.

Example 1

Preparation of high vapor transmission films containing siloxane without a base polymer

A reactor is charged under nitrogen with 35 g (0.0244 moles) polyethylene oxide, 100 g (0.0492 moles) polytetramethylene oxide, 1.5 g (0.0006 moles) polydimethylsiloxane/polyethylene oxide block copolymer, 320 ml. dimethylformamide and is heated to 45° C. Then 26.0 g (0.1040 moles) para, para'-diphenyl-methane diisocyanate is added and the mixture is maintained at 45 ± 3° C with agitation for 3 hrs. Heating is halted and the reactor is charged with 0.66 ml. (0.009 moles) ethylene diamine followed immediately by 355 ml. dimethylformamide, taking caution to control the exotherm below 50° C. At approximately 10 minute intervals, the remainder of the ethylene diamine is added according to the following schedule:

```
1st addition  (described above):   0.66 ml.
2nd addition: ..................   0.66 ml.
3rd addition: ..................   0.33 ml.
4th addition: ..................   0.33 ml.
                        Total ....  1.98 ml.
                        (0.0297 moles)
```

The reaction mixture is stirred for approximately one hour after the final addition. A film-forming, thermoplastic material is yielded with the following properties:

MVTR = 15.283 g/m²/24 hrs. for a 12,7 μm (0,5 mil) film
Young's modulus = 42,9 ± 2,7 kp/cm² (610 ± 38 psi)
Tensile strength = 317,3 kp/cm² (4514 psi)
Ultimate elongation (%) = 1085.

Example 2

Preparation of high vapor transmission films containing siloxane without a base polymer

A reactor is charged under nitrogen with 58.0 g (0.0405 moles) polyethylene oxide, 71.0 g (0.0349 moles) polytetramethylene oxide, 1.7 g (0.0007 moles) of a polydimethylsiloxane/polyethylene oxide block copolymer, and 250 ml. dimethylformamide, heated to 45 ± 3° C. Add 31.8 g (0.1272 moles), para, para'-diphenylmethane diisocyanate and maintained at 45° C with agitation for 3 hrs. Then 350 ml. dimethylformamide is added, followed by a solution of 3.42 ml. ethylene diamine dissolved in 100 ml. dimethylformamide gradually added over approximately a one-half hr. period. The reaction temperature is maintained to less than 50° C throughout the addition of chain extender. Following chain extension, the solution is stirred for an additional hour. This yields a film-forming material with the following properties:

6

EP 0 183 790 B1

MVTR = 36.243 g/m²/24 hrs. for a 12,7 μm (0,5 mil) film
Young's modulus = 83,9 ± 6,7 kp/cm² (1194 + 95 psi)
Tensile strength = 221,9 ± 12,4 kp/cm² (3156 + 176 psi)
Ultimate elongation (%) = 1046 ± 59
Young's modulus of a wet film (24 hrs. water immersion) = 60,3 ± 3,5 kp/cm² (858 ± 50 psi)
Tensile strength of a wet film = 174,6 ± 6,8 kp/cm² (2484 ± 97 psi)
Ultimate elongation (%) = 1204 ± 63

Example 3 (Comparison)

Preparation of high vapor transmission films without siloxane and without a base polymer

A nitrogen-purged reactor is charged with 57.9 g (0.0404 moles) polyethylene oxide, 72.8 g (0.0358 moles) polytetramethylene oxide, and 250 ml dimethylformamide and the temperature is raised to 45 ± 3° C. Then 31.8 g (0.1272 moles) para, para'-diphenylmethane diisocyanate is added and the mixture is agitated for 3 hours at 45° C.

Then 350 ml additional dimethylformamide is added followed by gradual addition of a solution of 3.07 g (0.0510 moles) ethylene diamine in 100 ml dimethylformamide over a one-half hour period. The solution temperature is maintained to less than 50° C throughout the reaction of the chain extender.

Following complete addition of the chain extender, the mixture is agitated for an additional hour. This yields a film-forming material with the following properties:
MVTR = 21.738 g/m²/24 hrs. for a 12,7 μm (0,5 mil) film
Young's modulus = 70,2 ± 4,7 kp/cm² (998 ± 67 psi)
Tensile strength = 240 ± 16 kp/cm² (3414 ± 228 psi)
Ultimate elongation (%) = 937 ± 30
Young's modulus of a wet film (24 hours water immersion) = 60 ± 4,2 kp/cm2 (854 ± 60 psi)
Tensile strength of a wet film = 227,2 ± 7,4 kp/cm² (3232 ± 105 psi)
Ultimate elongation (%) of a wet film = 1156 ± 89

Comparison of example 2 according to the invention and example 3 not according to the invention

|  | Example 2 | Example 3 |
|---|---|---|
| MVTR 12,7 μm film | 36.243 g/m²/24 hrs. | 21.738 g/m²/24 hrs. |
| Young's modulus | 83,9 ± 6,7 kp/cm² | 70,2 ± 4,7 kp/cm² |
| Tensile strength | 221,9 ± 12,4 kp/cm² | 240 ± 16 kp/cm² |
| Ultimate elongation | 1046 ± 59 % | 937 ± 30 % |

Example 4

Preparation of high vapor transmission films containing siloxane without a base polymer

A reactor under nitrogen atmosphere is charged with 39.7 g (0.0277 moles) polyethylene oxide, 38.2 g (0.0188 moles) polytetramethylene oxide, 1.6 g (0.0007 moles) of a polydimethylsiloxane/polyethylene oxide block copolymer, and 61.5 g (0.2458 moles) para, para'-diphenylmethane diisocyanate, and 200 ml. dimethylformamide, then heated to 42 ± 3° C. The temperature is maintained with agitation for 3 hours, then diluted with 60 additional ml. dimethylformamide.

Then 17.9 g (0.1987 moles) 1,4-butanediol in 75 ml. dimethylformamide is placed into an additional funnel and approximately 3/4 of this butanediol solution is added to the reaction mixture. After one hour, the remainder of the solution is added and the mixture is allowed to react for 2 additional hours. A film-forming material was yielded possessing the following characteristics:
MVTR = 7.618 g/m²/24 hrs. for a 12,7 μm (0,5 mil) film
Young's modulus = 275,2 ± 18 kp/cm² (3915 ± 257 psi)

7

Tensile strength = 308,5 kp/cm$^2$ (4388 psi)
Ultimate elongation (%) = 729 ± 31
Young's modulus of a wet film (24 hours water immersion) = 264,8 ± 9 kp/cm$^2$ (3767 ± 128 psi)
Tensile strength of a wet film = 252,5 ± 10,6 kp/cm$^2$ (3592 ± 151 psi)
Ultimate elongation (%) of a wet film = 756 ± 37

**Claims**

1. A moisture vapor permeable material in the form of a nonporous uniform film or coating of a thickness from 12,7 to 127 μm, said film or coating is obtained by using a solution of the material in dimethylacetamide, dimethylformamide, toluene/isopropanol or tetrahydrofuran and evaporating the solvent, said material comprising a segmented block multipolymer, said segmented multipolymer comprising an essentially linear hard segment copolymer chain characterized by at least one polyurethane or polyurethane urea hard segment and at least one copolymer soft block comprising polyoxytetramethylene oxide and polydialkylsiloxane as hydrophobic and polyethylene oxide as hydrophilic components.

2. A film according to claim 1 comprising said segmented block multipolymer in admixture with a base polymer, said segmented block multipolymer being present in the admixture in an amount to achieve a silicone concentration of at least 0,5% (by weight) and a polyethylene oxide concentration of at least 5 % (by weight), said film having a thickness of 12,7 to 50,8 μm.

3. A film according to claim 2, wherein said base polymer is a polyurethane polymer.

4. A film according to claim 3, wherein said base polymer is a polyurethane selected from polyether urethanurea, polyetherurethane and polyesterurethane.

5. A film according to claim 4, wherein said base polymer is a polyurethane urea formed by polymerization of diphenylmethane diisocyanate, ethylene diamine and polytetramethylene oxide.

6. A coated textile comprising a fabric web and a uniform nonporous coating on at least one surface of said web, said coating comprising a material according to one or more of the preceding claims 1 to 5.

**Revendications**

1. Matériau perméable à la vapeur humide, sous forme de pellicule ou revêtement homogène et non poreux, d'une épaisseur allant de 12,7 à 127 μm, ladite pellicule ou ledit revêtement étant obtenu en ce qu'on utilise une solution du matériau dans du diméthylacétamide, du diméthylformamide, du toluène/isopropanol ou du tétrahydrofuranne, et on évapore le solvant, ledit matériau comprenant un multipolymère-bloc segmenté, ledit multipolymère comprenant une chaîne de copolymères à segments durs essentiellement linéaire, caractérisé par au moins un segment dur de polyuréthane ou de polyuréthane-urée et au moins un bloc souple de copolymère comprenant de l'oxyde de polyoxytétraméthylène et un polydialkylsiloxane comme composants hydrophobes et de l'oxyde de polyéthylène comme composant hydrophile.

2. Pellicule selon la revendication 1, comprenant ledit multipolymère-bloc segmenté, mélangé à un polymère de base, ledit multipolymère-bloc segmenté étant contenu dans le mélange selon une quantité permettant d'obtenir une concentration de silicone d'au moins 0,5 % (en poids) et une concentration d'oxyde de polyéthylène d'au moins 5 % (en poids), ladite pellicule ayant une épaisseur de 12,7 à 50,8 μm.

3. Pellicule selon la revendication 2, caractérisée en ce que ledit polymère de base est un polymère de polyuréthane.

4. Pellicule selon la revendication 3, caractérisée en ce que ledit polymère de base est un polyuréthane choisi parmi une polyétheruréthane-urée, un polyétheruréthane et un polyesteruréthane.

5. Pellicule selon la revendication 4, caractérisée en ce que ledit polymère de base est une polyuréthane-urée formée par polymérisation de diphénylméthane-diisocyanate, d'éthylènediamine et d'oxyde de polytétraméthylène.

6. Textile enduit, comprenant une bande de tissu et un revêtement homogène et non poreux sur au moins une face de ladite bande de tissu, ledit revêtement comprenant un matériau selon l une ou plusieurs des revendications précédentes 1 à 5.

**Patentansprüche**

1. Wasserdampfdurchlässiges Material in Form eines nichtporösen einheitlichen Filmes oder Überzuges einer Dicke von 12,7 bis 127 $\mu$m, wobei der Film oder Überzug unter Verwendung einer Lösung des Materials in Dimethylacetamid, Dimethylformamid, Toluol/Isopropanol oder Tetrahydrofuran und Abdampfen des Lösungsmittels erhalten worden ist, wobei das Material aus einem segmentierten Block-Multipolymer besteht, und wobei dieses segmentierte Multipolymer eine im wesentlichen lineare Hart-Segment-Copolymer-Kette, welche durch wenigstens ein Polyurethan- oder Polyurethanharnstoff-Hart-Segment charakterisiert ist, und wenigstens einen Copolymer-Weich-Block enthält, der Polyoxytetramethylenoxid und Polydialkylsiloxan als hydrophobe und Polyethylenoxid als hydrophile Komponenten enthält.

2. Film nach Anspruch 1, dadurch gekennzeichnet, daß er das segmentierte Block-Multipolymer in Mischung mit einem Basis-Polymer enthält, wobei das segmentierte Block-Multipolymer in der Mischung in einer Menge enthalten ist, um eine Silikon-Konzentration von mindestens 0,5 Gew.-% und eine Polyethylenoxid-Konzentratiom von mindestens 5 Gew.-% zu erreichen, wobei der Film eine Dicke von 12,7 bis 50,8 $\mu$m hat.

3. Film nach Anspruch 2, dadurch gekennzeichnet, daß das Basis-Polymer ein Polyurethan-Polymer ist.

4. Film nach Anspruch 3, dadurch gekennzeichnet, daß das Basis-Polymer ein Polyurethan, ausgewählt aus der Gruppe Polyetherurethanharnstoff, Polyetherurethan und Polyesterurethan, ist.

5. Film nach Anspruch 4, dadurch gekennzeichnet, daß das Basis-Polymer ein Polyurethanharnstoff, hergestellt durch Umsetzung von Diphenylmethandiisocyanat, Ethylendiamin und Polytetramethylenoxid, ist.

6. Beschichtetes Textil, welches ein Gewebe und einen einheitlichen nicht-porösen Überzug auf wenigstens einer Oberfläche des Gewebes aufweist, wobei der Überzug aus einem Material entsprechend einem oder mehreren der vorhergehenden Ansprüche 1 bis 5 besteht.

9